# FASCICULE DE BREVET EUROPEEN

(11) **EP 2 785 747 B1**
(45) Date de publication et mention de la délivrance du brevet: **16.09.2015**
(21) Numéro de dépôt: 12753958.3
(22) Date de dépôt: 29.08.2012
(51) Int. Cl.: C08F 2/48

(54) **PROCEDE DE PREPARATION D'UNE MATRICE EN HYDROGEL PAR PHOTOPOLYMERISATION**
VERFAHREN ZUR HERSTELLUNG EINER HYDROGELMATRIX DURCH PHOTOPOLYMERISIERUNG
METHOD FOR PREPARING A HYDROGEL MATRIX BY PHOTOPOLYMERIZATION

(30) Priorité: 28.11.2011 FR 1160874
(43) Date de publication de la demande: 08.10.2014
(73) Titulaire: Commissariat à l'Énergie Atomique et aux Énergies Alternatives, 75015 Paris (FR); Centre National de la Recherche Scientifique (CNRS), 75016 Paris (FR)
(72) Inventeur: GULINO, Danielle, F-38660 Lumbin (FR); MGHARBEL, Abbas, F-38100 Grenoble (FR); NICOLAS, Alice, F-38120 Saint Egreve (FR)
(74) Mandataire: Ahner, Philippe
(86) Numéro de dépôt international: PCT/EP2012/066781
(87) Numéro de publication internationale: WO 2013/079231

(56) Documents cités:
- J.R.TSE, A.J. ENGLER: "Stiffness Gradients Mimicking In Vivo Tissue Variation Regulate Mesenchymal Stem Cell Fate", PLOS ONE, vol. 6, no. 1, E15978, 5 janvier 2011 (2011-01-05), pages 1-9, XP002679864,
- WONG JOYCE Y ET AL: "Directed movement of vascular smooth muscle cells on gradient-compliant hydrogels", LANGMUIR: THE ACS JOURNAL OF SURFACES AND COLLOIDS, AMERICAN CHEMICAL SOCIETY, WASHINGTON, DC, USA, vol. 19, no. 5, 1 janvier 2003 (2003-01-01), pages 1908-1913, XP002513302, ISSN: 0743-7463, DOI: 10.1021/LA026403P [extrait le 2003-01-09]
- J.R. TSE, A.J. ENGLER: "Preparation of Hydrogel Substrates with Tunable Mechanical Properties", CURRENT PROTOCOLS IN CELL BIOLOGY, 10.16, 1 juin 2010 (2010-06-01), pages 1-16, XP002679865,
- N. ZAARI, P. RAJAGOPALAN, S.K. KIM, A.J. ENGLER, AND J.Y. WONG: "PHOTOPOLYMERIZATION IN MICROFLUIDIC GRADIENT GENERATORS: MICROSCALE CONTROL OF SUBSTRATE COMPLIANCE TO MANIPULATE CELL RESPONSE", ADVANCED MATERIALS, vol. 16, no. 23-24, 17 décembre 2004 (2004-12-17), pages 2133-2137, XP002679866,
- KLOXIN A M ET AL: "In situ elasticity modulation with dynamic substrates to direct cell phenotype", BIOMATERIALS, ELSEVIER SCIENCE PUBLISHERS BV., BARKING, GB, vol. 31, no. 1, 1 janvier 2010 (2010-01-01), pages 1-8, XP026719454, ISSN: 0142-9612, DOI: 10.1016/J.BIOMATERIALS.2009.09.025 [extrait le 2009-09-27]

## Description

### DOMAINE TECHNIQUE

La présente invention a trait à un procédé de préparation d'une matrice en hydrogel par photopolymérisation, cette matrice pouvant être caractérisée par des zones distinctes contiguës pouvant présenter un gradient de rigidité de l'ordre du kPa/µm et une rigidité comprise entre 100 Pa et 500 kPa.

Ce procédé peut trouver application dans la conception de matrices destinées à être utilisées pour la discrimination de cellules, en particulier de cellules saines et cancéreuses.

Il est connu que les cellules interagissent avec leur environnement, qu'il soit cellulaire ou matriciel.

Trois facteurs essentiels influencent la survie cellulaire via leur capacité d'adhésion, de migration et de prolifération :
- les contraintes géométriques ;
- l'environnement chimique ; et
- l'environnement mécanique, tel que l'action de forces ou contraintes externes, les propriétés mécaniques de la matrice avec laquelle les cellules sont en contact.

Il est établi, notamment depuis les travaux de Lo et al. (Biophys.J., 79, 144-152) que les cellules sont sensibles aux propriétés élastiques de la matrice, sur laquelle elles se trouvent. Pour chaque type cellulaire, un seuil minimal de rigidité peut exister, au-dessous duquel la cellule ne parvient pas à adhérer malgré une chimie de surface favorable. Au-dessus de ce seuil, les cellules adhèrent et survivent. Il a été observé, notamment, que des cellules isolées .migrent vers les zones de la matrice où la rigidité est la plus élevée, cet effet étant d'autant plus marqué, que la matrice présente une rigidité voisine de celle correspondant à la rigidité seuil du type cellulaire considéré. Toutefois, les cellules cancéreuses présentent une réponse à la rigidité, qui diffère des cellules saines.

Sur la base du constat fait ci-dessus, il apparaît tout à fait envisageable d'effectuer une localisation, un guidage et/ou un tri cellulaire avec des matrices (ou substrats), dont les propriétés mécaniques sont modulées spatialement (par le biais de zones différentes présentant des rigidités distinctes) et dont les propriétés chimiques de surface sont uniformes.

Des matrices répondant à ces critères peuvent être des matrices polymériques du type hydrogel présentant une juxtaposition de zones de rigidités différentes. L'intérêt de ce type de matrices est multiple, en ce sens que :
- la préparation de ce type de matrices peut être plus simple que celle des puces chimiques ;
- le matériau constitutif de ces matrices se caractérise par une importante longévité (notamment, une absence de dégradation à l'échelle du mois) ;
- la possibilité d'une fonctionnalisation de surface sélective couplée aux motifs de rigidité.

Différentes tentatives d'élaboration de matrices du type hydrogel présentant une juxtaposition de zones de rigidités différentes ont été proposées dans l'art antérieur, et notamment par photopolymérisation par voie solution.

Ainsi, certains auteurs ont utilisé les outils de la microfluidique pour établir un gradient des constituants à réticuler, reléguant ainsi la technique de photopolymérisation à un intérêt uniquement pratique et non technologique. Les gradients de rigidité (de l'ordre du kPa/mm dans le meilleur des cas) sont limités par la microfluidique et non par la photopolymérisation.

D'autres auteurs ont choisi de photopolymériser une solution homogène à travers un masque, notamment dans le document Tse et al. (Curr.Protoc. Cell. Biol. 10.16.1-10.16.16, June 2010), les propriétés de la solution utilisée et les conditions opératoires permettant d'obtenir uniquement une durée de polymérisation de l'ordre de la minute et un gradient de rigidité de l'ordre du kPa/mm. Avec une telle durée de polymérisation, les espèces chimiques comprises dans la solution diffusent aisément d'une zone à l'autre au cours du processus de polymérisation, ce qui nuit à la résolution des zones formées et, qui plus est, génère une uniformisation de rigidité par diffusion de matière entre les zones plus éclairées et les zones moins éclairées lors de la photopolymérisation.

Au vu de ce qui existe dans le domaine de la préparation des matrices susmentionnées, les auteurs de la présente invention se sont proposé de mettre au point un procédé de préparation d'une matrice polymérique présentant des zones (ou motifs) de rigidité distinctes, permettant d'obtenir des zones de rigidité distinctes dans des durées de polymérisation inférieures à celles obtenues dans l'art antérieur, le gradient de rigidité entre deux zones contiguës pouvant être de l'ordre du kPa/µm.

J.R.Tse, A.J. Engler, PLOS ONE, vol. 6, no. 1, pages 1-9, décrit un procédé de préparation d'un hydrogel à gradient de rigidité par photopolymérisation qui est proche du présent procédé, mais diffère cependant en ce qu'aucun catalyseur de polymérisation n'est utilisé. Le gradient de rigidité est de l'ordre de 1kPa/ millimètre.

Wong et al, LANGMUIR, vol. 19, no. 5, pages 1908-1913, J.R. Tse, A.J. Engler, CURRENT PROTOCOLS IN CELL BIOLOGY, 10.16, pages 1-16, N. Zaari, P. Rajagopalan, S.K. Kim, A.J. Engler, J.Y. Wong, ADVANCED MATERIALS, vol. 16, no. 23-24, pages 2133-2137, et Kloxin et al, BIOMATERIALS, vol. 31, no. 1, pages 1-8 décrivent des procédés et des hydrogels similaires et ne décrivent également pas l'utilisation d'un catalyseur de polymérisation.

### EXPOSÉ DE L'INVENTION

Les auteurs ont découvert, de manière surprenante, qu'en utilisant dans la solution polymérique une combinaison d'ingrédients spécifiques, il est ainsi possible d'obtenir des matrices présentant les avantages mentionnées ci-dessus.

Ainsi, l'invention a trait à un procédé de préparation d'une matrice du type hydrogel comprenant au moins deux zones contiguës de rigidités distinctes, ledit procédé comprenant une étape de photopolymérisation d'une solution comprenant un ou plusieurs composés polymérisables et un initiateur de photopolymérisation, par application d'une lumière sur toute la solution et selon une intensité différente au niveau d'au moins deux zones de la solution, moyennant quoi il résulte une matrice d'hydrogel comprenant au moins deux zones contiguës de rigidités distinctes, caractérisé en ce que l'étape de photopolymérisation est réalisée en présence d'un catalyseur de polymérisation.

En jouant sur l'utilisation combinée d'un initiateur de photopolymérisation et d'un catalyseur de polymérisation dans la solution comprenant le ou les composés polymérisables et ainsi de la technique de photopolymérisation amorcée, l'on accède à une durée de polymérisation (qui peut être de l'ordre de la seconde) considérablement amoindrie par rapport au cas où la photopolymérisation est induite uniquement par l'utilisation simple d'un initiateur de photopolymérisation.

Cette propriété (à savoir, une durée de photopolymérisation amoindrie) se traduit par les avantages suivants :
- une limitation du phénomène de diffusion des réactifs (monomères, initiateur et catalyseur) d'une zone à l'autre lors du processus de photopolymérisation, ce qui permet, au final, l'obtention de zones de rigidités distinctes présentant une meilleure résolution ;
- la possibilité d'obtenir, en jouant sur une modulation spatiale de l'intensité à l'échelle de la largeur de zone que l'on souhaite obtenir (par exemple, de l'ordre de 1 µm), des zones étroites et contiguës de rigidités distinctes avec un gradient de rigidité entre deux zones très raide (par exemple, de l'ordre du kPa/µm).

Comme mentionné ci-dessus, il est fait usage, dans le cadre du procédé de l'invention, d'une solution comprenant un ou plusieurs composés polymérisables, un initiateur de photopolymérisation et un catalyseur de polymérisation, ceci n'excluant pas le fait que la solution puisse contenir d'autres ingrédients.

Il s'entend que le ou les composés polymérisables utilisés sont des composés précurseurs du matériau hydrogel, qui va constituer la matrice, à savoir un matériau du type gel obtenu en milieu aqueux, ces matériaux hydrogels pouvant être, par exemple :
- des polyacrylamides ;
- des polyéthylèneglycols comprenant des motifs répétitifs issus de la polymérisation de composés acrylates ou méthacrylates ;
- des polysaccharides, éventuellement modifiés (par exemple, des polysaccharides modifiés comprenant des motifs répétitifs issus de la polymérisation de composés acrylates) ;
- des (co)polymères issus de la polymérisation de composés diacrylates et/ou (méth)acrylates ;
- des alcools polyvinyliques comprenant des motifs répétitifs issus de la polymérisation de composés acrylates ou méthacrylates ;
- des dextranes comprenant des motifs répétitifs issus de la polymérisation de composés (méth)acrylates ;
- des polyfumarates de propylène et des dérivés de ceux-ci, comme des poly(fumarate de propylène-co-éthylèneglycol) ; et
- des combinaisons de ceux-ci.

Le ou les composés polymérisables peuvent être des monomères ou encore des oligomères ou polymères comportant au moins une fonction polymérisable.

Les composés polymérisables utilisés peuvent être ainsi :
- pour l'obtention de polyacrylamides, des monomères acrylamides ;
- pour l'obtention de polyéthylèneglycols comprenant des motifs répétitifs issus de la polymérisation de composés acrylates et/ou méthacrylates, des composé acrylates et/ou méthacrylates mis en présence de polyéthylèneglycols, la fonction polymérisable étant située au niveau de ces composés acrylates et/ou méthacrylates ;
- pour l'obtention de polysaccharides modifiés par des composés acrylates, des monomères acrylates (qui seront mis en présence de composés polysaccharides) ;
- pour l'obtention de (co)polymères issus de la polymérisation de composés diacrylates et/ou (méth)acrylates, des monomères diacrylates et/ou (méth)acrylates ;
- pour l'obtention d'alcools polyvinyliques comprenant des motifs répétitifs issus de la polymérisation de composés acrylates ou méthacrylates d'alcools polyvinyliques, des alcools polyvinyliques mis en présence de composés acrylates et/ou méthacrylates, la fonction polymérisable étant située au niveau de ces composés acrylates et/ou méthacrylates ;
- pour l'obtention des dextranes comprenant des motifs répétitifs issus de la polymérisation de composés (méth)acrylates, des monomères (méth)acrylates mis en présence de composés dextranes ;
- pour l'obtention de polyfumarates de propylène et des dérivés de ceux-ci, des monomères fumarates de propylène.

Les initiateurs de photopolymérisation susceptibles d'être utilisés peuvent être des initiateurs de la famille des cétones aromatiques, des composés acridines, des composés fluorones.

A titre d'exemples de cétones aromatiques, on peut mentionner :
- les cétals benzyliques, tels que ceux répondant à la formule suivante : 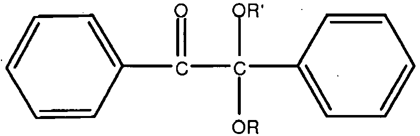 dans lesquels R et R' peuvent représenter un groupe alkyle ;
- des composés benzoines ;
- des cétones α-aminoaromatiques ;
- des composés phosphines ;
- des composés benzophénones ;
- de la thioxanthone.

Comme composés phosphines, on peut mentionner les composés vendus sous la dénomination Irgacure, tel que l'Irgacure 819^{®}, de formule suivante : 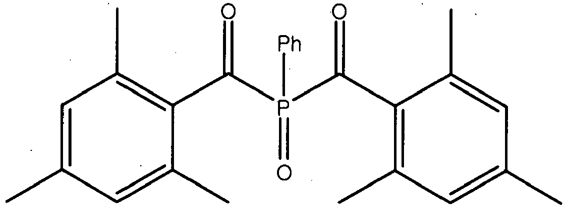 Ph désignant un groupe phényle.

Les catalyseurs de polymérisation, qui sont responsables de la forte accélération de la vitesse de réaction de photopolymérisation, sont avantageusement des composés amines.

Lorsque l'initiateur de photopolymérisation présente une grande réactivité vis-à-vis du ou des composés polymérisables présents en solution, un composé du type amine primaire ou amine secondaire peut être suffisant. Dans ce cas, le composé amine réagit avec un initiateur de photopolymérisation pour former un radical libre, ce dernier réagissant avec l'oxygène par une réaction de peroxydation, le composé amine pouvant ainsi être considéré comme jouant le rôle indirect de piège à oxygène (ce dernier étant un frein à une réaction radicalaire en chaîne).

Lorsque l'initiateur de photopolymérisation présente une efficacité moyenne vis-à-vis du ou des composés polymérisables, le composé amine peut être un intermédiaire réactionnel entre l'initiateur de photopolymérisation et le ou les composés polymérisables, remplissant ainsi un rôle de catalyseur. Dans ce cas, le composé amine est avantageusement un composé du type amine tertiaire.

A titre d'exemple de composé amine susceptible d'assurer le rôle de catalyseur de polymérisation, on peut citer la n-propylamine.

Outre les ingrédients susmentionnés (monomère(s), initiateur et catalyseur), la solution peut comprendre :
- des colorants (tels que du bleu de méthylène), de sorte à ajuster la longueur d'onde de la lampe de l'initiateur de photopolymérisation ;
- des agents de réticulation ;
- de l'eau, de préférence, désionisée ; et
- les mélanges de ceux-ci.

Comme agents de réticulation, on peut citer des monomères comprenant au moins deux fonctions polymérisables, par exemple, des monomères bisacrylamides, tels que le N,N'-méthylènebis-acrylamide.

A titre d'exemple, la solution comprend de l'acrylamide, un initiateur de photopolymérisation de formule suivante : 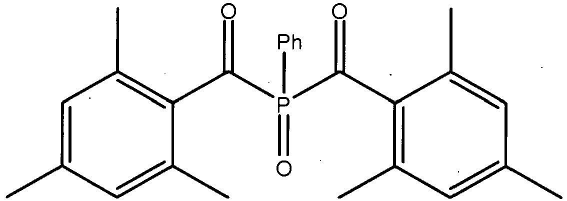 Ph désignant un groupe phényle,
de la n-propylamine en tant que catalyseur de polymérisation, de la N,N'-méthylènebisacrylamide en tant qu'agent de réticulation et de l'eau.

Comme mentionné ci-dessus, il est appliqué une lumière sur toute la solution (pour engendrer une photopolymérisation de l'intégralité de la solution en hydrogel) et selon une intensité différente au niveau d'au moins deux zones contiguës de la solution, moyennant quoi il résulte une matrice d'hydrogel comprenant au moins deux zones contiguës de rigidités distinctes.

En d'autres termes, cela signifie que l'on applique une lumière, par exemple, une lumière UV, et plus spécifiquement, une lumière UV-A, à la solution selon une distribution d'intensité modulée spatialement, de sorte à accéder à la répartition souhaitée de zones contiguës de rigidités distinctes (sachant que, selon l'intensité lumineuse appliquée, cela influe sur le taux de réticulation de l'hydrogel et donc sur sa rigidité).

La distribution d'intensité modulée spatialement peut être obtenue par :
(i) l'utilisation, au-dessus de la solution à traiter, d'un masque, par exemple un masque comprenant un ou plusieurs niveaux de gris, dont l'opacité reflète le taux de réticulation souhaité ; ou
(ii) une source lumineuse modulée spatialement en intensité par un réseau d'interférences, un réseau de diffraction ou un motif holographique.

Lorsqu'il est utilisé un masque, le matériau constitutif de ce masque est choisi en fonction de :
(i) l'absorption du matériau absorbant dans la plage de longueur d'ondes de la lampe utilisée lors de la photopolymérisation ;
(ii) la résolution des zones (ou motifs) souhaitées.

Lorsqu'il s'agit de réaliser des motifs de taille submicronique, le masque peut consister :
- en un substrat en verre ou quartz recouvert selon une disposition appropriée à l'obtention des motifs souhaités de couches de métaux ou oxydes métalliques ;
- en un substrat en cristaux liquides ;
- en un substrat en matériau thermoplastique (tel qu'un polyméthacrylate, un polycarbonate, un polystyrène) obtenu par thermomoulage ou gravure ionique.

Lorsqu'il s'agit de réaliser des motifs de taille de 10 µm ou plus, le masque peut consister en un film plastique traité par impression laser ou en un assemblage de films opaques ou réflectants.

Lorsqu'il s'agit de réaliser des motifs plus complexes, il s'entend qu'il faudra utiliser des modulations d'intensité plus complexes que des modulations simplement binaires, qui peuvent être mises en oeuvre grâce à l'utilisation d'un masque comportant une répartition de niveaux de gris nécessaire à l'obtention des motifs souhaités. Ce type de masque peut consister en une plaque de verre ou quartz recouverte, sur une face, par une couche métallique (par exemple, en chrome), qui est lithographiée et/ou gravée de sorte à créer différents niveaux de gris.

De préférence, la lumière est appliquée sur la solution via un masque, de sorte à délimiter des zones d'intensité lumineuse distinctes.

Dans ce cas, le procédé de l'invention peut comprendre une étape préalable de préparation du masque.

En fonction de la matrice en hydrogel souhaitée, l'homme du métier pourra être amené à procéder à des opérations préliminaires avant d'effectuer l'étape de photopolymérisation en tant que telle.

Les opérations préliminaires peuvent être les suivantes :
(i) le choix des ingrédients constitutifs de la solution, comme les composés polymérisables (dont le choix sera fixé par le matériau hydrogel que l'on souhaite obtenir), l'initiateur de photopolymérisation, qui peut être choisi en fonction de la longueur d'onde de la source lumineuse et la résolution spatiale des motifs de rigidité souhaitée et le catalyseur de polymérisation, qui peut être choisi en fonction de la nature de l'initiateur de photopolymérisation ;
(ii) l'évaluation de la durée seuil pour polymériser la solution par la source lumineuse, sachant que plus le gradient de rigidité souhaité est fort (par exemple, de 1 kPa/µm), plus la durée seuil doit être faible (par exemple, de l'ordre de la seconde), ce qui peut amener à devoir adapter la concentration en initiateur et/ou catalyseur pour accélérer ou ralentir la réaction de photopolymérisation en fonction du gradient de rigidité souhaité ;
(iii) la caractérisation de la rigidité en fonction de la puissance transmise et de la durée pour une illumination homogène, ce qui peut constituer un repère pour le choix des paramètres d'application de la lumière lors de la photopolymérisation (ces paramètres pouvant être aussi qualifiés de paramètres d'illumination) ;
(iv) à partir de la caractérisation susmentionnée, la sélection du contraste d'illumination entre les motifs rigides et les motifs dits « mous » (en comparaison aux motifs rigides), cette sélection pouvant passer par le choix des niveaux de gris pour les masques, le choix du gradient d'intensité pour les motifs holographiques.

La photopolymérisation est réalisée, avantageusement en atmosphère inerte (par exemple, azote ou argon) de sorte à éviter la présence d'oxygène, qui inhibe la réaction de polymérisation. Il est ainsi possible, en plus de travailler en atmosphère inerte, de procéder au dégazage de la solution, de sorte à éliminer l'oxygène présent dans cette solution.

La photopolymérisation est conduite, classiquement, à une température où la solution reste liquide, à savoir à une température s'échelonnant classiquement entre 0 et 100°C.

Comme évoqué plus haut, les matrices préparées selon le procédé de l'invention peuvent se caractériser par au moins deux zones contiguës de rigidités distinctes présentant un gradient de rigidité égal à 1 kPa/µm, avantageusement, une rigidité comprise en 100 Pa et 500 kPa et, avantageusement, une largeur de zones de l'ordre de 1 µm.

Plus spécifiquement, une desdites deux zones contiguës présentant la rigidité la plus élevée se présente sous la forme d'un motif présentant une dimension inférieure à 100 µm.

Des matrices de ce type peuvent être en un hydrogel issu de la polymérisation de l'acrylamide et du N,N'-méthylènebisacrylamide.

Du fait que ces matrices peuvent permettre des sauts de rigidité de plusieurs kPa sur une échelle inférieure à la taille d'une cellule biologique, c'est donc tout naturellement que ces matrices peuvent trouver application dans un procédé d'adhésion cellulaire, ce procédé pouvant avoir pour visée le confinement de cellules, le tri de cellules, la localisation de cellules, et plus spécifiquement la localisation de cellules avec contrôle de la géométrie cellulaire, la déformation de cellules. De ce fait, ces matrices peuvent être utilisées comme outils de diagnostic pour la détermination des organes cibles des cellules métastasiques, les cellules présentant une réponse à la rigidité différente selon leurs types.

Pour ce faire, en vue d'être utilisées pour ces applications, les matrices peuvent être amenées à subir, en fin de procédé de préparation après l'étape de photopolymérisation, une étape de fonctionnalisation de la matrice par une protéine induisant une adhésion cellulaire *via* les intégrines, une telle protéine pouvant être de la fibronectine, du collagène, de la laminine ou des peptides du type RGD.

L'invention peut ainsi avoir trait à un procédé d'adhésion cellulaire *in vitro* comprenant :
a) une étape de mise en oeuvre du procédé de préparation d'une matrice de type hydrogel tel que défini ci-dessus ;
b) une étape de dépôt sur la matrice obtenue à l'étape a) de cellules d'un ou plusieurs types, moyennant quoi les cellules d'au moins un des types susmentionnés adhérent en tout ou partie à certaines zones de la matrice de rigidité plus élevée que les zones contiguës.

Ce procédé d'adhésion peuvent avoir pour visée de :
- localiser les cellules sur des zones spécifiques ;
- plus spécifiquement, localiser sur des zones spécifiques avec contrôle de la géométrie cellulaire, ce qui revient à dire que le procédé a trait à la mise en forme de cellules ; et
- déformer des cellules.

Ainsi, plus spécifiquement, l'invention peut aussi avoir trait à un procédé de mise en forme cellulaire *in vitro* comprenant :
a) une étape de mise en oeuvre du procédé de préparation d'un matrice de type hydrogel tel que défini ci-dessus;
b) une étape de dépôt sur la matrice obtenue à l'étape a) de cellules d'un ou plusieurs types, moyennant quoi les cellules d'au moins un des types susmentionnés se concentrent, en tout ou partie sur certaines zones de la matrice de rigidité plus élevée que les zones contiguës et adoptent la forme desdites zones de rigidité plus élevée.

Les zones de rigidité plus élevée que leurs zones contiguës sont qualifiées ci-dessous de motifs, ces motifs constituant des zones d'attraction cellulaire.

Par adhésion cellulaire, on précise que l'on entend, classiquement, une localisation cellulaire totale ou partielle sur lesdites zones, ce qui n'exclut pas que ces cellules puissent être mobiles au sein desdites zones, dans le cas, notamment, où ces zones présenteraient un gradient de rigidité.

Concernant la localisation de cellules de façon générale, les matrices obtenues selon le procédé de l'invention peuvent permettre de localiser les cellules, par exemple, de type épithéliale, endothéliale, fibroblastique, astrocyte ou musculaire, ces cellules ayant pour particularité de migrer vers les zones dont la rigidité est la plus élevée. Il peut être envisageable, par exemple, de concevoir des matrices comportant des zones de rigidité organisées de telle sorte à constituer des routes à cellules, à savoir, par exemple, des canaux dont la rigidité est supérieure aux zones adjacentes à ces canaux, permettant ainsi une concentration des cellules dans ces canaux. Qui plus est, s'il est prévu un gradient de rigidité à l'intérieur même des canaux, les cellules migreront dans la direction de la rigidité croissante.

Il peut être envisageable, également, de concevoir des matrices comportant des zones de rigidité plus élevée (ces zones pouvant être qualifiées de motifs) que les zones environnant ces motifs, ces motifs présentant au moins une dimension cellulaire (par exemple, inférieure à 100 µm, et plus spécifiquement, de l'ordre de 10 à 100 µm suivant le type cellulaire) et, qui plus est, ces motifs étant espacés de façon à :
(i) limiter le nombre de cellules présentes sur les zones environnant les motifs, ces zones présentant une rigidité moindre que lesdits motifs ; et
(ii) interdire que les cellules se partagent entre plusieurs motifs.

A titre d'exemple, pour des types cellulaires du type REF52 et HUVEC, un espacement entre motifs allant de 80 à 200 µm peut permettre de souscrire aux conditions (i) et (ii) susmentionnées.

Concernant la localisation de cellules avec contrôle de la géométrie cellulaire, les matrices obtenues par le procédé de l'invention peuvent permettre, grâce aux gradients de rigidité, une migration des cellules vers les zones de plus grande rigidité et en conférant à ces zones des formes de motifs spécifiques, une conformation de la forme des cellules ainsi positionnées à la forme de motifs desdites zones, ces motifs présentant au moins une dimension de l'ordre de l'échelle cellulaire (par exemple un dimension allant de 10 à 100 µm suivant le type cellulaire), de sorte à permettre le confinement dans des conditions géométriques et mécaniques identiques. Il peut être notamment possible de créer une matrice reproduisant un ensemble de conditions proches des conditions d'adhésion rencontrées *in vivo* dans les tissus ou les embryons.

De ce fait, la localisation d'une grande quantité de cellules, isolées ou en agrégats de taille contrôlée, dans des conditions géométriques et mécaniques identiques et notamment dans des conditions géométriques et mécaniques se rapprochant de celles rencontrées dans les tissus de l'organisme peut être un outil de choix pour tester l'impact de molécules d'intérêt susceptibles d'agir sur le fonctionnement cellulaire, car il permet une analyse statistique sur une large population en une expérience unique. L'analyse peut, par exemple, se porter sur l'analyse de la polarité cellulaire par rapport à l'administration de molécules d'intérêt, la polarité, par exemple, dans un tissu épithélial ayant des conséquences sur l'intégrité du tissu (un-défaut dans la polarité baso-latérale des cellules rénales pouvant entraîner la formation de kystes et de fibroses rénales) ou encore un impact sur la morphogénèse, l'extension des tissus embryonnaires se faisant dans la direction allongée des cellules. Connaître l'impact de la molécule d'intérêt sur la polarité cellulaire ou inversement l'impact de la polarité cellulaire sur l'efficacité de la molécule d'intérêt se révèlent des paramètres cruciaux pour évaluer l'efficacité d'une molécule d'intérêt ou ses effets indésirables potentiels. Plus généralement, peuvent être testés l'impact de la molécule d'intérêt sur l'étalement cellulaire, l'organisation du cytosquelette, le maintien (ou non) de la sensibilité cellulaire aux propriétés mécaniques du substrat, associée à un dysfonctionnement de l'adhésion cellulaire. Et inversement, l'impact de la position du centrosome, de l'appareil de Golgi et du noyau (contrôlées par la contrainte géométrique) et de la contractibilité cellulaire (contrôlée par l'élasticité de la matrice extracellulaire) sur l'effet de la molécule d'intérêt.

Concernant la déformation de cellules, les matrices obtenues selon le procédé de l'invention peuvent permettre de déformer substantiellement des cellules. Il peut être envisageable, par exemple, de concevoir des matrices comportant des zones de rigidité plus élevée que les zones contiguës auxdites zones. Plus spécifiquement, la matrice peut présenter un ensemble de motifs présentant une rigidité plus élevée que les zones entourant lesdits motifs. Ces motifs présentent, avantageusement, au moins une dimension d'échelle sous-cellulaire (par exemple, une dimension allant de 0,5 à 10 µm) et présentent, avantageusement, un espacement permettant un étalement des cellules sur plusieurs motifs, cet étalement étant à l'origine de la déformation cellulaire. D'un point de vue fonctionnel, les adhésions focales et les fibres de stress associées sont favorisées sur les zones de plus forte rigidité et les complexes focaux et les filaments d'actine enchevêtrés associés sont favorisés sur les zones de plus faible rigidité.

Concernant le tri de cellules, les matrices obtenues selon le procédé de l'invention peuvent permettre d'effectuer le tri, par exemple, entre des cellules gliales et des neurones. Les cellules gliales sont le support mécanique et chimique de la croissance des neurones dans le cerneau. Actuellement, les études *in vitro* sur des neurones primaires sont limitées par la difficulté de gérer la présence des cellules gliales. La présence de ces cellules permet de reproduire un environnement chimique proche des conditions *in vivo* mais rend très délicat l'organisation spatiale des neurones, car ces derniers poussent sur le tapis de cellules gliales. Sachant que les cellules gliales ne poussent pas sur des substrats de faible rigidité, il peut être envisageable avec des matrices présentant des zones rigides et des zones plus rigides de réaliser la culture concomitante de neurones et de cellules gliales avec une ségrégation de ces deux types de cellules, moyennant quoi l'on pourrait obtenir l'organisation d'un réseau de neurones « purs » dans un environnement chimique de cellules gliales.

Le tri cellulaire peut être basé également selon la mobilité des cellules au sein de zones de plus forte rigidité, les cellules les moins mobiles se déplaçant moins au sein desdites zones de plus forte rigidité.

Concernant la détermination des organes cibles des cellules métastasiques, il est connu que certaines tumeurs disséminent des cellules métastasiques dans des organes lointains, comme celles du sein ou des poumons, qui se disséminent dans les poumons, les reins, les os ou le cerveau. Sachant que les taux de prolifération et de migration des cellules cancéreuses sont maximaux sur des matrices au niveau des zones de rigidité correspondant à celle de l'invention, l'on pourrait envisager de concevoir, conformément au procédé de l'invention, des matrices contenant des bandes de rigidité choisies en fonction des phénotypes attendus (le phénotype cellulaire dominant dans chacune des bandes étant celui visant l'organe, qui présente cette même rigidité).

Les matrices de l'invention peuvent être incorporées dans des dispositifs destinés à recevoir des cellules, plus particulièrement, des cellules eucaryotes, de préférence, des cellules de mammifères, et de préférence encore, des cellules humaines, lesquels dispositifs comprennent une matrice telle que définie ci-dessus et obtenue par le procédé tel que défini ci-dessus.

L'invention va être, à présent, décrite à la lumière d'un exemple de mise en oeuvre du procédé conforme à l'invention, cet exemple n'étant fourni qu'à titre d'illustration de l'invention et n'en constituant en aucun cas une limitation.

### BRÈVE DESCRIPTION DES DESSINS

La figure 1 représente une vue du dispositif mis en oeuvre dans le cadre de l'exemple 1.
La figure 2a représente un graphique illustrant l'évolution de la hauteur h (en µm) de la matrice obtenue pour l'exemple 1 en fonction de la distance d (en µm) (0 correspondant à la délimitation entre la zone illuminée à travers la zone transparente du masque et la zone illuminée à travers la zone de niveau de gris).
La figure 2b représente un graphique illustrant l'évolution de la rigidité Y (en kPa) de la matrice obtenue pour l'exemple 1 en fonction de la distance d (en µm) (0 correspondant à la délimitation entre la zone illuminée à travers la zone transparente du masque et la zone illuminée à travers la zone de niveau de gris).
La figure 2c représente une photographie de la matrice obtenue pour l'exemple 1, après ensemencement cellulaire.
La figure 3 représente une vue du dispositif mis en oeuvre dans le cadre de l'exemple 2.
La figure 4 représente un graphique illustrant l'évolution de la rigidité Y (en kPa) de la matrice obtenue pour l'exemple 2 en fonction de la distance d (en µm).
La figure 5 représente une photographie de la matrice obtenue pour l'exemple 2, après ensemencement cellulaire.
La figure 6 représente une vue du dispositif mis en oeuvre dans le cadre de l'exemple 3.
La figure 7 représente un graphique illustrant l'évolution de la rigidité Y (en kPa) de la matrice obtenue à l'exemple 3 en fonction d'une distance d (en µm) par rapport au centre du canal de cette matrice (cette distance au centre ayant pour valeur 0 au niveau de l'abscisse).
La figure 8 représente une photographie de la matrice obtenue pour l'exemple 3, après ensemencement cellulaire.
La figure 9 représente une vue du dispositif mis en oeuvre dans le cadre de l'exemple 4.
La figure 10 représente un graphique illustrant l'évolution de la rigidité Y (en kPa) de la matrice obtenue pour l'exemple 4 en fonction de la distance d (en µm).
La figure 11 représente une photographie de la matrice vue de dessus obtenue pour l'exemple 4.
La figure 12 représente une vue du dispositif mis en oeuvre dans le cadre de l'exemple 5.
La figure 13 est une photographie illustrant le phénomène de localisation cellulaire avec conformation de la géométrie cellulaire à celle du motif de rigidité plus élevée que les zones environnant ce motif conformément à l'exemple 5.
La figure 14 représente deux vues (a et b) agrandies de la matrice vue de dessus après ensemencement cellulaire dans le cadre de l'exemple 6.
La figure 15 représente une vue agrandie (avec des inserts à droite et à gauche pris à différentes durées d'ensemencement cellulaire) de la matrice vue de dessus après ensemencement cellulaire dans le cadre de l'exemple 7.
La figure 16 représente une vue agrandie de la matrice vue de dessus après ensemencement cellulaire dans le cadre de l'exemple 8.
La figure 17 représente une vue agrandie de la matrice vue de dessus après ensemencement cellulaire dans le cadre de l'exemple 9.

### EXPOSÉ DÉTAILLÉ DE MODES DE RÉALISATION PARTICULIERS

### EXEMPLE 1

Cet exemple illustre la préparation d'une matrice en hydrogel comportant deux zones de rigidité distinctes impliquant les étapes suivantes :
- une étape de préparation d'une lamelle de verre destinée à servir de support à la matrice en hydrogel (a) ;
- une étape de préparation d'un masque (b) ;
- une étape de préparation de la matrice en hydrogel (c).

### a) Préparation de la lamelle

Une lamelle de verre basale est nettoyée avec une solution de soude à 0,1 mol/L pendant 10 minutes. Elle est ensuite rincée intensivement à l'eau puis à l'éthanol. On l'immerge ensuite pendant 5 minutes dans une solution d'APTMS (3-aminopropyltriméthoxysilane) à 10% dans l'éthanol (5ml d'APTMS pour 45mL d'éthanol), sous agitation lente. La lamelle est ensuite rincée à l'éthanol puis laissée pendant 10 minutes sous agitation lente dans un bain d'éthanol. Après un rinçage simple à l'eau désionisée, la lamelle est immergée dans une solution de glutaraldéhyde à 0,5% dans l'eau pendant 25 minutes. Elle est ensuite rincée intensivement à l'eau et séchée fortement. On obtient ainsi une lamelle de verre présentant des fonctions aldéhydes à sa surface, qui vont permettre le greffage covalent de l'hydrogel en polyacrylamide, qui va être préparé subséquemment.

### b) Préparation d'un masque

Une lame de microscopie optique (26 mm x 76 mm) est lavée dans une solution d'eau oxygénée/acide sulfurique concentré en proportion 1:1, pendant 20 minutes. Une plaque de silicium clivée est fixée sur la moitié gauche de la lame. Elle sert à cacher la partie transparente avant de procéder au dépôt. Ensuite 1nm de titane puis 4 nm de chrome sont déposés à l'aide d'un évaporateur à canon d'électrons de type Plassys.

La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

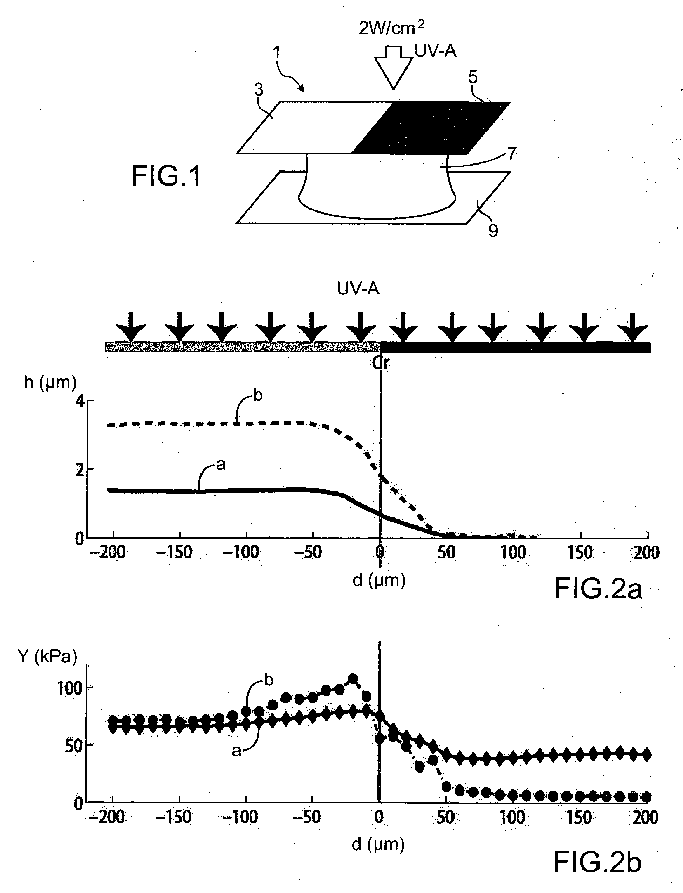

La lame obtenue est représentée sur la figure 1 par la référence 1, cette lame comprenant deux zones :
- une zone transparente 3 ;
- une zone 5 couverte d'une couche de 5 nm (1 nm de titane et 4 nm de chrome) (dite zone de niveau de gris), contiguë à la zone transparente,
cette lame constituant un masque destiné à être interposé entre un flux lumineux (indiqué par une flèche sur la figure indiquant qu'il s'agit d'un flux UV-A à 2W/cm²) et la solution (représentée par la référence 7) déposée sur la lame de verre 9 susmentionnée et destinée à être photoréticulée en vue de constituer la matrice visée, à savoir une matrice présentant deux zones contiguës présentant une rigidité distincte, ces zones correspondant à la géométrie de celle du masque.

Un deuxième masque est préparé de la même manière, que celle définie ci-dessus, si ce n'est que la zone dite de niveau de gris est constituée d'une couche de 1 nm de titane, sur laquelle est déposée 19 nm de chrome.

### c) Préparation de la matrice en hydrogel

De l'Irgacure 819^{®} (1,7 mg) est pesé dans un flacon opaque aux rayons ultraviolets. On y ajoute de la n-propylamine (10 µL). L'ensemble est chauffé à 50°C pendant 2 minutes. Après chauffage, on obtient une solution homogène et transparente. De l'eau désionisée (490 µL), de l'acrylamide (250 µL d'une solution à 40%) et du N,N'-méthylènebisacrylamide (250 µL d'une solution à 2%) sont ajoutés. L'ensemble est homogénéisé doucement à la pipette, pour limiter l'incorporation d'oxygène.

30 µL de la solution obtenue sont déposés sur la lamelle de verre de 30 mm préparée selon le protocole explicité ci-dessus. La lamelle est placée sur un porte-échantillon présentant des cales d'épaisseur, qui permettent de maintenir un espacement de 40 µm entre la lamelle et le masque préparé préalablement, déposé sur les cales d'épaisseur.

L'ensemble (masque, solution, lamelle) est illuminé à l'aide d'une lampe fibrée Eleco UVP281 (2W/cm²) pendant 20 secondes, tel que cela est illustré sur la figure 1. Cet ensemble est ensuite plongé dans l'eau pour détacher le masque de la matrice en hydrogel ainsi formée à l'aide d'une pince. La matrice en hydrogel est rincée 3 fois avec de l'eau désionisée.

Deux essais ont été effectués selon les mêmes modalités :
- un premier essai avec le masque comportant une zone de niveau de gris (constituée par une couche de 1 nm de titane et 4 nm de chrome) ; et
- un second essai avec le masque comportant une zone de niveau de gris (constituée par une couche de 1 nm de titane et 19 nm de chrome).

Pour ces deux essais, il a été procédé à une détermination de la topographie de surface ainsi que de la rigidité de la matrice obtenue.

Pour déterminer la topographie de surface, il a été procédé à la mesure de la hauteur de la matrice en fonction de la distance d par rapport à la délimitation entre la zone illuminée à travers la zone transparente (cette délimitation ayant pour valeur 0 au niveau de l'abscisse) et la zone illuminée à travers la zone de niveau de gris du masque, les résultats étant reportés sur la figure 2a, qui représente l'évolution de la hauteur h (en µm) en fonction de la distance d (en µm), cette distance étant explicitée dans la partie supérieure du graphique par la représentation des zones du masque au travers desquelles a été pratiquée l'illumination.

Que ce soit pour le premier essai (courbe a) ou pour le second essai (courbe b), l'on constate une diminution substantielle de la hauteur de la matrice dès lors que l'on passe de la zone illuminée à travers la zone transparente du masque à la zone illuminée à travers la zone de niveau de gris du masque.

Pour déterminer la rigidité de la matrice obtenue, il a été procédé à la mesure de la rigidité locale de l'hydrogel par microscopie à force atomique (AFM) en milieu aqueux (marque JPK). La résistance de la matrice à l'enfoncement d'une pointe est enregistrée. Un scan de 400 µm est effectué à travers la frontière. Les scans sont réalisés avec un pas de 10µm. Il en résulte une série de courbes d'indentation. Chaque courbe est traitée selon le protocole du fabricant avec un modèle d'indentation élastique. Chaque point du graphe est la moyenne de 3 mesures réalisées sur 3 points espacés de 10 µm dans une direction parallèle à la frontière.

Les résultats sont reportés sur la figure 2b représentant l'évolution de la rigidité Y (en kPa) en fonction de distance d (en µm) par rapport à la délimitation entre la zone illuminée à travers la zone transparente (cette délimitation ayant pour valeur 0 au niveau de l'abscisse) et la zone illuminée à travers la zone de niveau de gris du masque.

Que ce soit pour le premier essai (courbe a) ou le second essai (courbe b), il ressort qu'il y a une diminution substantielle de la rigidité, dès lors que l'on passe de la zone illuminée à travers la zone transparente du masque (cette zone illuminée pouvant être qualifiée de zone rigide) à la zone illuminée à travers la zone de niveau de gris du masque (cette zone illuminée pouvant être qualifiée de zone molle), le gradient de rigidité pouvant être de l'ordre du kPa/µm, notamment pour le second essai.

Il a également été procédé à une culture cellulaire sur les matrices obtenues ci-dessus.

De façon préalable, la matrice est fonctionnalisée avec de la fibronectine. Pour ce faire, la lamelle de verre de diamètre 30 mm, sur laquelle est polymérisé l'hydrogel, est collée au fond d'une boîte de Pétri percée de diamètre 35 mm. L'eau est retirée de la boîte, 1mg de Sulfo SDA (Pierce) (correspondant à un succinimidylester diazirine) est dissous dans 2,27 mL d'eau désionisée stérile. La solution obtenue est déposée sur la matrice et laissée 2 heures dans le noir. Après ce temps, elle est aspirée à la pipette et 2 mL d'une solution de fibronectine à 25 mg/mL dans du PBS stérile est déposée pendant 1 heure. La boîte est toujours conservée dans le noir. Cette dernière solution est aspirée à la pipette et la boîte est placée sous la lampe Eleco UVP281 (2 W/cm²) pendant 3 min. La matrice est rincée 3 fois avec du PBS (sel tampon phosphate) stérile.

Des cellules HUVEC (qui correspondent à des cellules endothéliales humaines matures isolées à partir de la veine de cordon ombilical) sont ensemencées à une densité de 2000 cellules/cm² dans le milieu M199, 20% FCS (sérum foetal de veau), 2% LSGS (qui est un supplément de croissance à faible teneur en sérum), 1% ATAM (antibiotiques (pénicilline, streptomycine) + antimycotique, Invitrogen).

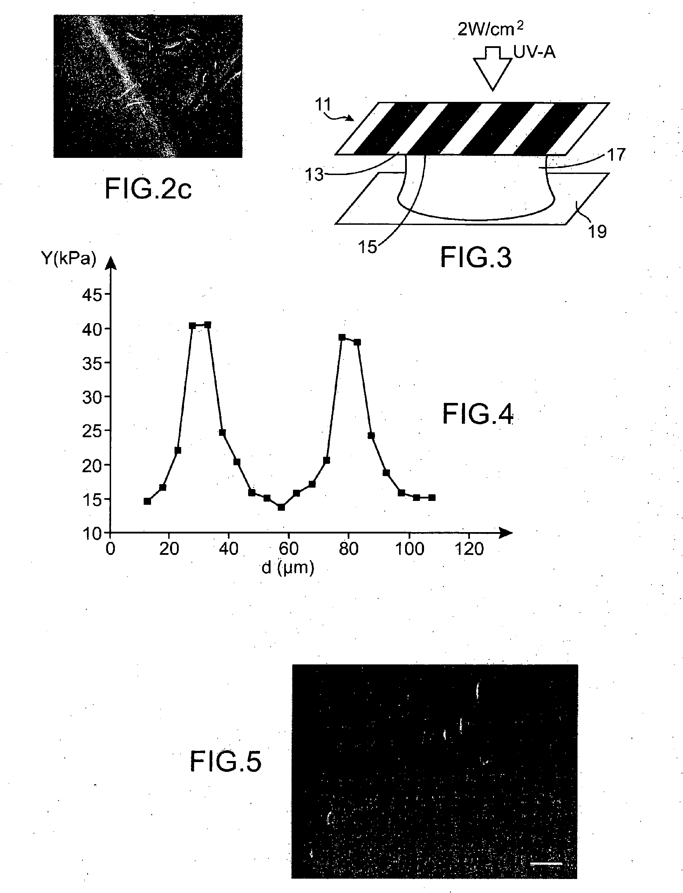

Comme il ressort de la figure 2c, qui est une photographie faite par microscopie à contraste de phase, avec un agrandissement 20x sur un microscope inversé 24 heures après l'ensemencement, il y traversée d'une cellule de la délimitation entre la zone molle et la zone rigide.

Le profil topographique n'influence pas l'organisation cellulaire. Les cellules traversent la frontière de rigidité en se positionnant perpendiculairement à la frontière (comme illustré sur la figure 2c). Dans le cas où la topographie influencerait le comportement cellulaire, les cellules devraient s'aligner sur la frontière, ce qui n'est manifestement pas le cas.

### EXEMPLE 2

Cet exemple illustre la préparation d'une matrice comportant des zones de rigidités distinctes sous forme de lignes de 10 µm alternées avec des lignes de 40 µm impliquant les étapes suivantes :
- une étape de préparation d'une lamelle de verre destinée à servir de support à la matrice (a) ;
- une étape de préparation d'un masque (b) ;
- une étape de préparation de la matrice en hydrogel (c)

### a) Préparation de la lamelle

La lamelle est préparée de la même façon que, dans l'exemple 1 explicité ci-dessus.

### b) Préparation d'un masque

Une lame de microscopie optique (26 mm x 76 mm) est lavée dans une solution d'eau oxygénée/acide sulfurique concentré en proportion 1:1, pendant 20 minutes. Sur cette lame, 1 nm de titane puis 9 nm de chrome sont déposés à l'aide d'un évaporateur à canon d'électrons de type Plassys. Une résine du type AZ1512HS (disponible chez Clariant) diluée à 50% dans son solvant AZ-EB (qui est un solvant propylèneglycolmonométhyléther acétate) est déposée sur la lame côté chromé à l'aide d'une tournette, moyennant quoi l'on obtient une épaisseur de résine de 600nm. Elle est recuite 2 minutes à 120°C. Elle est illuminée à travers un masque Sodalime présentant le motif désiré pendant 2 secondes (aligneur MJB4 à 30 mW/cm²). Après développement (AZ-Developer + eau désionisée 1:1, 1 minute), la lame est rincée intensivement à l'eau désionisée.

Puis la lame est placée dans un réacteur de gravure de type DPS, et gravée pendant 30 secondes à l'aide d'un traitement au chlore (2/3 Cl₂:1/3 O₂) sous une pression entre 10 et 25 mTorr. La résine est retirée par un plasma O₂ de 30 secondes dans le réacteur DPS. La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

Le masque est représenté sur la figure 3 par la référence 11, ce masque comprenant une alternance entre des zones transparentes 13 (non recouvertes de titane et de chrome) consistant en des lignes de 10 µm de largeur et des zones 15 consistant en des lignes de 40 µm de largeur couvertes d'une couche de 10 nm d'épaisseur (1 nm de titane et 9 nm de chrome) (ces zones pouvant être qualifiées de zones de niveau de gris).

Ce masque est destiné à être interposé entre un flux lumineux (indiqué par une flèche sur la figure indiquant qu'il s'agit d'un flux UV-A à 2 W/cm²) et la solution (représentée par la référence 17) déposée sur la lame de verre 19 susmentionnée et destinée à être photoréticulée en vue de constituer la matrice visée, à savoir une matrice présentant des zones contiguës présentant une rigidité distincte, à savoir des lignes de 10 µm de largeur alternées avec des lignes de 40 µm de largeur.

### c) Préparation de la matrice en hydrogel

La matrice en hydrogel est préparée de la même façon que, dans l'exemple 1 explicité ci-dessus.

Il a été procédé à une détermination de la rigidité de la matrice obtenue.

Pour ce faire, la rigidité locale du gel est mesurée à l'aide d'un microscope à force atomique en milieu aqueux (marque JPK). La résistance du gel à l'enfoncement de la pointe est enregistrée. Un scan de 100 µm est effectué à partir du centre du réseau de lignes espacées de 40 µm. Les scans sont réalisés avec un pas de 5 µm. Il en résulte une série de courbes d'indentation. Chaque courbe est traitée selon le protocole du fabricant avec un modèle d'indentation élastique. Chaque point du graphe est la résultante de moyenne de mesure sur deux courbes.

Les résultats sont reportés sur la figure 4 représentant l'évolution de la rigidité Y (en kPa) en fonction d'une distance d (en µm). Le point d'abscisse 0 correspond approximativement au centre du réseau.

L'allure de la courbe atteste de la présence de canaux de rigidité distants d'environ 40 µm, ces canaux de rigidité correspondant aux zones illuminées à travers les zones transparentes du masque susmentionné.

Il a également été procédé à une culture cellulaire sur les matrices obtenues ci-dessus.

De façon préalable, la matrice est fonctionnalisée avec de la fibronectine. Pour ce faire, la lamelle de verre de diamètre 30 mm, sur laquelle est polymérisé l'hydrogel, est collée au fond d'une boîte de Pétri percée de diamètre 35 mm. L'eau est retirée de la boîte, 1 mg de Sulfo SDA (Pierce) (correspondant à un succinimidylester diazirine) est dissous dans 2,27 mL d'eau désionisée stérile. La solution obtenue est déposée sur la matrice et laissée 2 heures dans le noir. Après ce temps, elle est aspirée à la pipette et 2 mL d'une solution de fibronectine à 25 mg/mL dans du PBS stérile est déposée pendant 1 heure. La boîte est toujours conservée dans le noir. Cette dernière solution est aspirée à la pipette et la boîte est placée sous la lampe Eleco UVP281 (2 W/cm²) pendant 3 min. La matrice est rincée 3 fois avec du PBS stérile.

Des cellules LN229 (cellules tumorales de glioblastome extraites du cortex frontal droit pariéto-occiputal) sont ensemencées à une densité de 5000 cellules/cm² dans un milieu DMEM (milieu minimum essentiel de Eagle modifié par Dubelcco) complété avec 10% FBS et 1% ATAM.

Comme il ressort de la figure 5, qui est une photographie faite par microscopie à contraste de phase, avec un agrandissement 20x sur un microscope inversé 2 heures après l'ensemencement, il y a concentration des cellules le long des zones illuminées à travers les zones transparentes du masque, ces zones correspondant aux zones rigides (et correspondant aux canaux de rigidité susmentionnés).

### EXEMPLE 3

Cet exemple illustre la préparation d'une matrice comportant des zones de rigidités distinctes impliquant les étapes suivantes :
- une étape de préparation d'une lamelle de verre destinée à servir de support à la matrice (a) ;
- une étape de préparation d'un masque (b) ;
- une étape de préparation de la matrice en hydrogel (c).

### a) Préparation de la lamelle

La lamelle est préparée de la même façon que, dans l'exemple 1 explicité ci-dessus.

### b) Préparation d'un masque

Une lame de microscopie optique (26 mm x 76 mm) est lavée dans une solution d'eau oxygénée/acide sulfurique concentré en proportion 1:1, pendant 20 minutes. Sur cette lame, une résine du type AZ1512HS (disponible chez Clariant) est déposée à l'aide d'une tournette à raison de 2500 tours/min pendant 120 secondes. Elle est recuite 2 minutes à 100°C. Elle est illuminée 35 secondes avec une lampe UV de 6 mW/cm² filtrée sur la longueur d'onde 365 nm à travers un masque PDF imprimé sur un transparent et présentant une ligne noir de 100 µm de largeur. Le motif est développé à l'aide du développeur AZ-EBR pendant 30 secondes. Après développement, la lame est de nouveau recuite 2 minutes à 120°C. 1 nm de titane puis 4 nm de chrome sont déposés à l'aide d'un évaporateur à canon d'électrons de type Plassys. Ensuite la lame est immergée 1 minute dans l'acétone, afin de retirer la ligne de résine. Il en résulte un canal de 100 µm transparent au milieu de la lame chromée.

La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

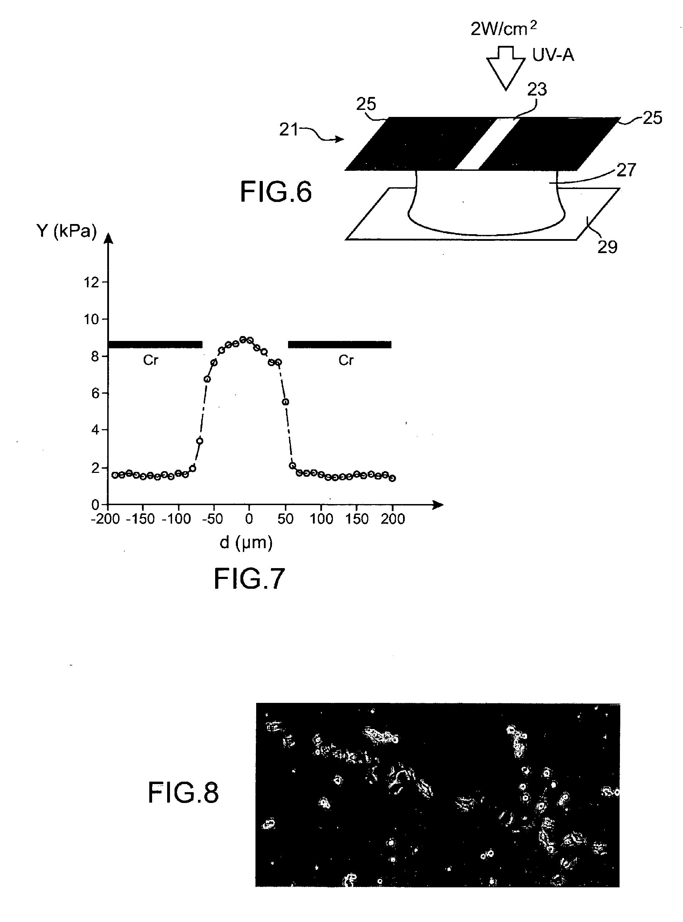

Le masque obtenu est représenté sur la figure 6 par la référence 21, ce masque comprenant une zone transparente 23 (non recouvertes de titane et de chrome) consistant en une ligne de 100 µm de largeur et deux zones 25 encadrant la zone 23, ces deux zones étant couvertes d'une couche de 5 nm d'épaisseur (1 nm de titane et 4 nm de chrome) (ces zones pouvant être qualifiées de zones de niveau de gris).

Ce masque est destiné à être interposé entre un flux lumineux (indiqué par une flèche sur la figure indiquant qu'il s'agit d'un flux UV-A à 2 W/cm²) et la solution (représentée par la référence 27) déposée sur la lame de verre 29 susmentionnée et destinée à être photoréticulée en vue de constituer la matrice visée, à savoir une matrice présentant des zones contiguës présentant une rigidité distincte, à savoir, dans ce cas, une zone centrale rigide encadrées par deux zones d'extrémité molles.

### c) Préparation de la matrice en hydrogel

La matrice en hydrogel est préparée de la même façon que, dans l'exemple 1 explicité ci-dessus, si ce n'est que la durée d'illumination n'est que de 8 s.

Il a été procédé à une détermination de la rigidité de la matrice obtenue.

Pour ce faire, la rigidité locale du gel est mesurée à l'aide d'un microscope à force atomique en milieu aqueux (marque JPK). La résistance du gel à l'enfoncement de la pointe est enregistrée. Un scan de 200 µm est effectué à travers le canal. Les scans sont réalisés avec un pas de 10 µm. Il en résulte une série de courbes d'indentation. Chaque courbe est traitée selon le protocole du fabricant avec un modèle d'indentation élastique. Chaque point du graphe est la résultante de moyenne de deux mesures effectuées sur deux points distincts de 10 µm parallèlement au canal.

Les résultats sont reportés sur la figure 7 représentant l'évolution de la rigidité Y (en kPa) en fonction de la distance d (en µm) par rapport au centre du canal (cette distance au centre ayant pour valeur 0 au niveau de l'abscisse).

Au regard de l'allure de la courbe, il ressort l'existence d'un canal de rigidité correspondant à la zone illuminée à travers la zone transparente du masque.

Il a également été procédé à une culture cellulaire sur les matrices obtenues ci-dessus.

De façon préalable, la matrice est fonctionnalisée avec de la fibronectine. Pour ce faire, la lamelle de verre de diamètre 30 mm, sur laquelle est polymérisé l'hydrogel, est collée au fond d'une boîte de Pétri percée de diamètre 35 mm. L'eau est retirée de la boîte, 1 mg de Sulfo SDA (Pierce) (correspondant à un succinimidylester diazirine) est dissous, dans 2,27 mL d'eau désionisée stérile. La solution obtenue est déposée sur la matrice et laissée 2 heures dans le noir. Après ce temps, elle est aspirée à la pipette et 2 mL d'une solution de fibronectine à 25 µg/mL dans du PBS stérile est déposée pendant 1 heure. La boîte est toujours conservée dans le noir. Cette dernière solution est aspirée à la pipette et la boîte est placée sous la lampe Eleco UVP281 (2 W/cm²) pendant 3 min. La matrice est rincée 3 fois avec du PBS stérile.

Des cellules Hela (cellules immortelles extraites d'une tumeur maligne du col de l'utérus) sont ensemencées à une densité de 5000 cellules/cm² dans un milieu DMEM complété avec 10% FBS et 1% ATAM.

Comme il ressort de la figure 8, qui est une photographie faite par microscopie à contraste de phase, avec un agrandissement 20x sur un microscope inversé 2 heures après l'ensemencement, il y a concentration des cellules le long du canal susmentionné, ce canal correspondant à la zone de rigidité la plus élevée de la matrice.

### EXEMPLE 4

Cet exemple illustre la préparation d'une matrice comportant des zones de rigidités distinctes se présentant sous forme d'un premier réseau de lignes de 6 µm de largeur alternées avec des lignes de 2 µm de largeur et d'un deuxième réseau de lignes de 4 µm de largeur alternées avec des lignes de 2 µm de largeur impliquant les étapes suivantes :
- une étape de préparation d'une lamelle de verre destinée à servir de support à la matrice (a) ;
- une étape de préparation d'un masque (b) ;
- une étape de préparation de la matrice en hydrogel (c).

### a) Préparation de la lamelle

La lamelle est préparée de la même façon que, dans l'exemple 1 explicité ci-dessus.

### b) Préparation d'un masque

Une lame de microscopie optique (26 mm x 76 mm) est lavée dans une solution d'eau oxygénée/acide sulfurique concentré en proportion 1:1, pendant 20 minutes. Sur cette lame, 1 nm de titane puis 4 nm de chrome sont déposés à l'aide d'un évaporateur à canon d'électrons de type Plassys. Une résine du type AZ1512HS (disponible chez Clariant) diluée à 50% dans son solvant AZ-EBR (qui est un solvant propylèneglycolmonométhyléther acétate) est déposée sur la lame côté chromé à l'aide d'une tournette, moyennant quoi l'on obtient une épaisseur de résine de 600nm. Elle est recuite 2 minutes à 120°C. Elle est illuminée à travers un masque Sodalime présentant le motif désiré pendant 2 secondes (aligneur MJB4 à 30 mW/cm²). Après développement (AZ-Developer + eau désionisée 1:1, 1 minute), la lame est rincée intensivement à l'eau désionisée.

Puis la lame est placée dans un réacteur de gravure de type DPS, et gravée pendant 30 secondes à l'aide d'un traitement au chlore (2/3 Cl₂:1/3 O₂) sous une pression entre 10 et 25 mTorr. La résine est retirée par un plasma O₂ de 30 secondes dans le réacteur DPS. La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

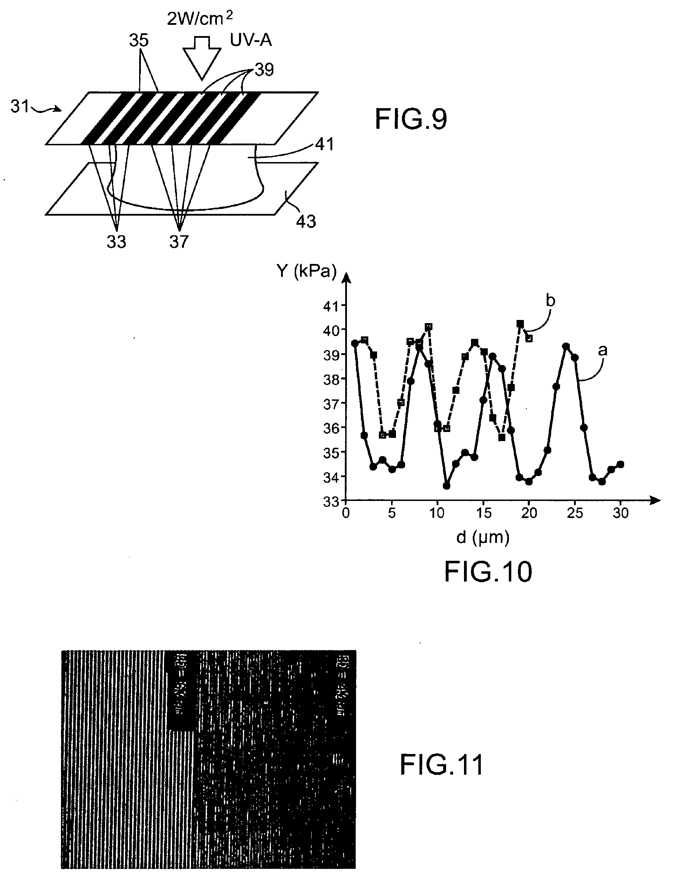

Le masque est représenté sur la figure 9 par la référence 31, ce masque comprenant un premier réseau de lignes 33 de 6 µm de largeur couvertes (dites lignes de niveau de gris) alternées avec des lignes 35 de 2 µm de largeur (lignes transparentes) et d'un deuxième réseau de lignes 37 de 4 µm de largeur couvertes (dites lignes de niveau de gris) alternées avec des lignes 39 de 2 µm de largeur (lignes transparentes, c'est-à-dire non recouvertes de titane et de chrome).

Ce masque est destiné à être interposé entre un flux lumineux (indiqué par une flèche sur la figure indiquant qu'il s'agit d'un flux UV-A à 2 W/cm²) et la solution (représentée par la référence 41) déposée sur la lame de verre 43 susmentionnée et destinée à être photoréticulée en vue de constituer la matrice visée, à savoir une matrice présentant un premier réseau de lignes de 6 µm de largeur (lignes molles) alternées avec des lignes de 2 µm de largeur

(lignes rigides) et d'un deuxième réseau de lignes de 4 µm de largeur (lignes molles) alternées avec des lignes de 2 µm de largeur (lignes rigides).

### c) Préparation de la matrice en hydrogel

La matrice en hydrogel est préparée de la même façon que, dans l'exemple 1 explicité ci-dessus.

Il a été procédé à une détermination de la rigidité de la matrice obtenue.

Pour ce faire, la rigidité locale du gel est mesurée à l'aide d'un microscope à force atomique en milieu aqueux (marque JPK). La résistance du gel à l'enfoncement de la pointe est enregistrée. Un scan de 30 µm est effectué à partir du centre du réseau de lignes espacées de 6 µm. De manière identique, un scan de 20 µm est effectué à partir du centre du réseau de lignes espacées de 4 µm. Les scans sont réalisés avec un pas de 1 µm. Il en résulte une série de courbes d'indentation. Chaque courbe est traitée selon le protocole du fabricant avec un modèle d'indentation élastique. Chaque point du graphe est la moyenne de deux mesures effectuées sur deux points distants de 10 µm parallèlement aux lignes.

Les résultats sont reportés sur la figure 10 représentant l'évolution de la rigidité Y (en kPa) en fonction d'une distance d (en µm). L'origine d=0 sur chacun des courbes correspond aux centres des réseaux de lignes espacées respectivement de 4 µm (pointillés gris) et de 6 µm (ligne continue noire).

Deux courbes sont représentées : une courbe a) pour le premier réseau de lignes (alternance de lignes molles de 6 µm alternées avec des lignes rigides de 2 µm) et une courbe b (alternance de lignes molles de 4 µm alternées avec des lignes rigides de 2 µm).

L'allure de ces deux courbes atteste de la présence de canaux de rigidité localisés au niveau des lignes rigides, ces canaux de rigidité étant espacées de 6 µm (courbe a) ou 4 µm (courbe b), le gradient de rigidité étant très important notamment pour le premier réseau de lignes entre lignes rigides et lignes molles, cette disposition étant attestée par la figure 11, représentant une photographie de la matrice obtenue vue de dessus.

### EXEMPLE 5

Cet exemple illustre la préparation d'une matrice en hydrogel comportant des zones de rigidité distinctes comportant respectivement, de la gauche vers la droite, les motifs suivants :
- deux lignes adjacentes comportant chacune trois motifs en forme d'ancres
- deux lignes adjacentes comportant chacune trois motifs en forme de triangles ; et
- deux lignes adjacentes comportant chacune trois motifs en forme de bonnets d'âne,
ledit procédé impliquant les étapes suivantes :
- une étape de préparation d'une lamelle de verre destinée à servir de support à la matrice en hydrogel (a) ;
- une étape de préparation d'un masque (b) ;
- une étape de préparation de la matrice en hydrogel (c).

### a) Préparation de la lamelle

La lamelle est préparée de la même façon que, dans l'exemple 1 explicité ci-dessus.

### b) Préparation d'un masque

Une lame de microscopie optique (26 mm x 76 mm) est lavée dans une solution d'eau oxygénée/acide sulfurique concentré en proportions 1:1, pendant 20 minutes. Sur cette lame, 1 nm de titane puis 9 nm de chrome sont déposés à l'aide d'un évaporateur à canon d'électrons de type Plassys. Une résine du type AZ1512HS (disponible chez Clariant) diluée à 50% dans son solvant AZ-EBR (qui est un solvant propylèneglycolmonométhyléther acétate) est déposée sur la lame côté chromé à l'aide d'une tournette à raison de 3000 tours/min pendant 30 secondes, moyennant quoi l'on obtient une épaisseur de résine de 600 nm. Elle est illuminée à travers un masque Sodalime présentant les motifs désirés. Après développement, la lame est placée dans un réacteur de gravure de type DPS, et gravée pendant 30 secondes à l'aide d'un traitement au chlore (2/3 Cl₂:1/3 O₂) sous une pression entre 10 et 25 mTorr. La résine est retirée par un plasma O₂ de 30 secondes dans le réacteur DPS. La lame est ensuite rendue hydrophobe par un traitement Optool (Daikin DSX) : immersion pendant 1 minute dans l'Optool dilué à 1/1000 dans du perfluorohexane. Puis la lame est laissée 1 heure dans de la vapeur d'eau à 80°C. Enfin, elle est immergée sous agitation lente pendant 10 minutes dans du perfluorohexane.

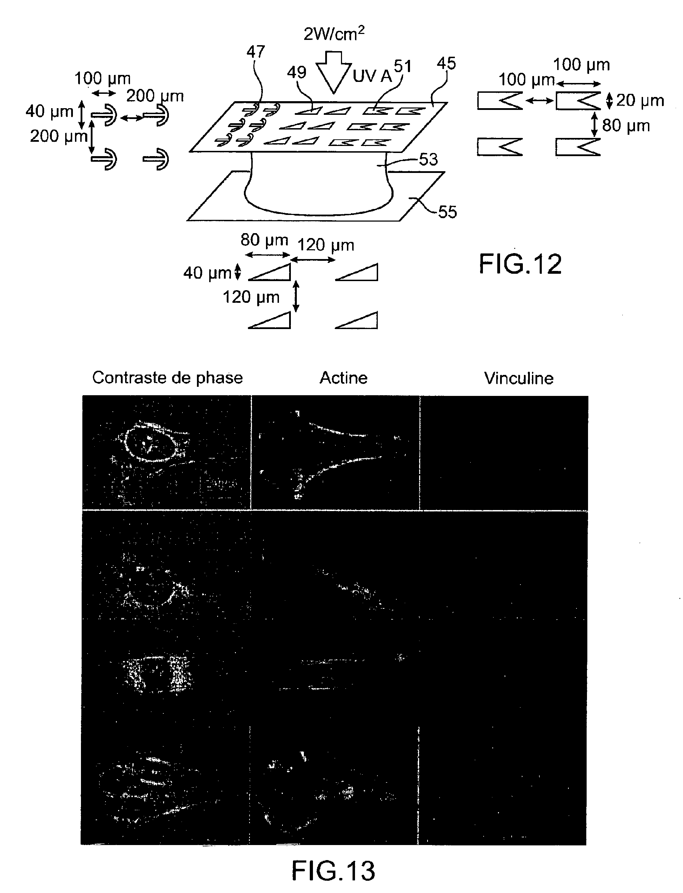

Le masque est représenté sur la figure 12 par la référence 45, ce masque comprenant, de la gauche vers la droite, un premier réseau de lignes 47 comprenant chacune trois motifs en forme d'ancre (dont les dimensions et l'espacement sont indiqués à gauche du masque), un deuxième réseau de lignes 49 comprenant chacune trois motifs en forme de triangles rectangles (dont les dimensions et l'espacement sont indiqués dans la partie inférieure de la figure) et un troisième réseau de lignes 51 comportant chacune trois motifs en forme de bonnets d'âne (dont les dimensions et l'espacement sont indiqués à droite du masque), les motifs du premier réseau, du deuxième réseau et du troisième réseau étant transparents, c'est-à-dire non recouverts de titane et de chrome.

Ce masque est destiné à être interposé entre un flux lumineux (indiqué par une flèche sur la figure indiquant qu'il s'agit d'un flux UV-A à 2 W/cm²) et la solution (représentée par la référence 53) déposée sur la lame de verre 55 susmentionnée et destinée à être photoréticulée en vue de constituer la matrice visée, à savoir une matrice présentant un premier réseau de lignes comprenant chacune trois motifs en forme d'ancre, un deuxième réseau de lignes comprenant chacune trois motifs en forme de triangles rectangles et un troisième réseau de lignes comportant chacune trois motifs en forme de bonnets d'âne.

### c) Préparation de la matrice en hydrogel

La matrice en hydrogel est préparée de la même façon que, dans l'exemple 1 explicité ci-dessus.

Il a été procédé à une culture cellulaire sur les matrices obtenues ci-dessus.

Des cellules REF52 sont ensemencées d'une manière homogène sur la matrice susmentionnée à raison d'une densité cellulaire de 5000 cellules/cm². Deux heures après l'ensemencement, les cellules sont fixées à l'aide de paraformaldéhyde (symbolisé par PAF) à 4% contenant 0,5 % de triton pendant 10 minutes puis avec du PAF à 4% pendant 20 minutes. Sur les cellules ainsi fixées et perméabilisées, l'actine est marquée à la phaloïdine-Cy5, la vinculine avec un anticorps monoclonal anti-vinculine puis avec un anticorps anti-IdG de souris couplé à l'Alexa 488, tandis que les noyaux le sont avec le colorant Hoestch. Après ces marquages fluorescents, l'observation des cellules est faite en microscopie à contraste de phase et de fluorescence en utilisant un objectif *60 sur un microscope inversé.

Comme illustré par la figure 13, les cellules se sont localisées sur les motifs susmentionnés de rigidité plus élevée (c'est-à-dire les motifs en forme d'ancres, de triangles rectangles et de bonnets d'âne).

### EXEMPLE 6

Cet exemple illustre la préparation d'une matrice en hydrogel comportant des zones de rigidité distinctes comportant un réseau de plots de diamètre de 2 µm espacés de 6 µm ou un réseau de plots de diamètre de 5 µm espacés de 7,5 µm, ces plots ayant une rigidité de 15 kPa, tandis que l'espace entre les plots est constitué d'une matrice molle ayant une rigidité de 6 kPa.

### a) Préparation de la lamelle

La lamelle est préparée de la même façon que, dans l'exemple 1 explicité ci-dessus.

### b) Préparation d'un masque

Le masque est préparé de la même façon que dans les exemples mentionnés ci-dessus.

### c) Préparation de la matrice en hydrogel

La matrice en hydrogel est préparée de la même façon que les exemples précédents.

Il a été procédé à une culture cellulaire sur les matrices obtenues ci-dessus.

Des cellules REF52 surexprimant la paxiline couplée à la YFP (symbolisées par REF52-Pax-YFP) sont ensemencées d'une manière homogène sur la matrice susmentionnée à raison d'une densité cellulaire de 5000 cellules/cm². Deux heures après l'ensemencement, les cellules sont fixées à l'aide de paraformaldéhyde (symbolisé par PAF) à 4% contenant 0,5 % de triton pendant 10 minutes puis avec du PAF à 4% pendant 20 minutes. Sur les cellules ainsi fixées et perméabilisées, l'actine est marquée à la phaloïdine-Cy5, la vinculine avec un anticorps monoclonal anti-vinculine puis avec un anticorps anti-IgG de souris couplé à l'Alexa 488, tandis que les noyaux le sont avec le colorant Hoestch. Après ces marquages fluorescents, l'observation des cellules est faite en microscopie à contraste de phase et de fluorescence en utilisant un objectif *60 sur un microscope inversé.

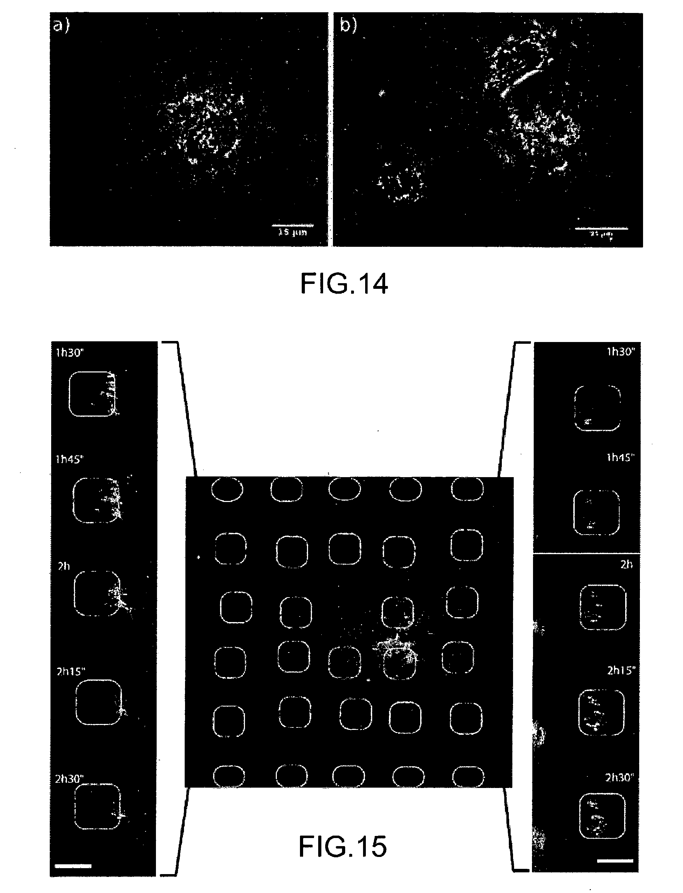

Comme illustré sur la figure 14, illustrant une vue agrandie des matrices (partie (a) pour la matrice à plots de diamètre de 2 µm ou partie (b) pour la matrice à plots de diamètre de 5 µm), l'adhérence des cellules se fait au niveau des contacts focaux, où se rassemble la vinculine, ces contacts focaux s'élaborant uniquement sur les plots rigides (c'est-à-dire les zones de plus forte rigidité). Quand la distance entre plots s'accroît, la forme cellulaire devient de plus en plus contrainte par la position des contacts focaux.

### EXEMPLE 7

Cet exemple 7 est une variante de l'exemple 6 susmentionnée réalisée dans des conditions similaires, si ce n'est que la matrice comporte des plots ayant une rigidité de 12 kPa et un diamètre de 6 µm, ces plots étant espacés de 9 µm, l'espace entre les plots étant constitué d'une matrice molle ayant une rigidité de 4 kPa.

Les images ont été prises toutes les quinze minutes comme illustré sur la figure 15 jointe en annexe. L'adhérence des cellules se fait au niveau des contacts focaux, qui s'établissent uniquement au niveau des plots rigides, là où se rassemble la paxiline-YFP.

Les inserts de gauche et de droite présentent l'évolution au cours du temps de deux contacts focaux. Le contact focal dans l'insert de gauche se désagrège progressivement, alors que celui dans l'insert de droite se renforce. Ces effets se traduisent par une migration cellulaire de la gauche vers la droite.

### EXEMPLE 8

Cet exemple vise à mettre en avant la modulation de la forme cellulaire en variant l'espacement des plots rigides.

Il constitue une variante de l'exemple 6 susmentionné réalisé dans des conditions similaires, si ce n'est que la matrice comporte des plots ayant une rigidité de 6 kPa et un diamètre de 10 µm, ces plots étant espacés de 10 µm, l'espace entre les plots étant constitué d'une matrice molle ayant une rigidité de 1 kPa (valeur de rigidité inférieure au seuil de survie cellulaire)

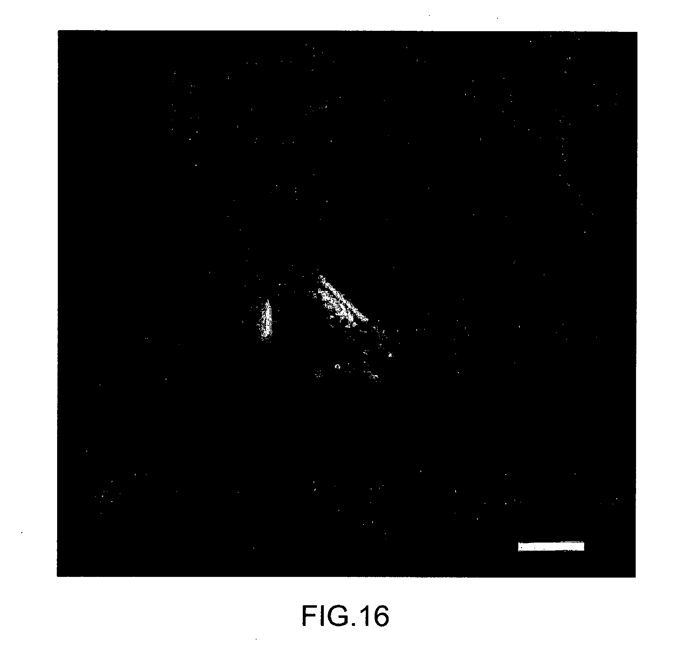

Comme illustré sur la figure 16, une cellule REF52 adopte une forme triangulaire fixée par la répartition des plots. Il est à noter que seules les cellules ayant directement été déposées sur les motifs ou à une distance inférieure à une taille cellulaire (de l'ordre de 10 µm) ont survécu et se sont organisées sur les plots rigides.

### EXEMPLE 9

Cet exemple constitue une variante de l'exemple 6 susmentionné réalisée dans des conditions similaires, si ce n'est que la matrice comporte des plots ayant une rigidité de 15 kPa et un diamètre de 3 µm, ces plots étant espacés de 1 µm, l'espace entre les plots étant constitué d'une matrice molle ayant une rigidité de 5 kPa.

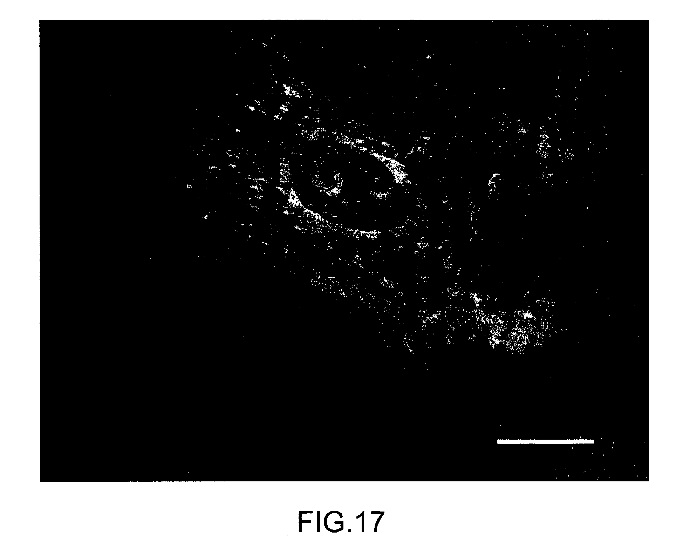

Comme illustré sur la figure 17, une cellule adopte une forme totalement déformée.

Des exemples 7 à 9 susmentionnés, qui illustrent des matrices à motifs rigides de taille sous-cellulaire, il peut en être déduit que la taille des motifs limite la croissance des sites adhésifs et la densité des motifs influence la géométrie cellulaire. De ce fait, le choix de leur taille et de leur espacement influence, d'une part, la géométrie de la cellule et, d'autre part, les forces contractiles appliquées par la cellule.

## Revendications

1. Procédé de préparation d'une matrice du type hydrogel comprenant au moins deux zones contiguës de rigidités distinctes comprenant une étape de photopolymérisation d'une solution comprenant un ou plusieurs composés polymérisables et un initiateur de photopolymérisation, par application d'une lumière sur toute la solution et selon une intensité différente au niveau d'au moins deux zones de la solution, moyennant quoi il résulte une matrice d' hydrogel comprenant au moins deux zones contiguës de rigidités distinctes, **caractérisé en ce que** l'étape de photopolymérisation est réalisée en présence d'un catalyseur de polymérisation.

2. Procédé selon la revendication 1, dans lequel la matrice du type hydrogel est en un matériau choisi parmi :
- des polyacrylamides ;
- des polyéthylèneglycols comprenant des motifs répétitifs issus de la polymérisation de composés acrylates ou méthacrylates ;
- des polysaccharides, éventuellement modifiés ;
- des (co)polymères issus de la polymérisation de composés diacrylates et/ou (méth)acrylates ;
- des alcools polyvinyliques comprenant des motifs répétitifs issus de la polymérisation de composés acrylates ou méthacrylates ;
- des dextranes comprenant des motifs répétitifs issus de la polymérisation de composés (méth)acrylates ;
- des polyfumarates de propylène et des dérivés de ceux-ci ; et
- des combinaisons de ceux-ci.

3. Procédé selon la revendication 1 ou 2, dans lequel l'initiateur de photopolymérisation est choisi parmi les cétones aromatiques, les composés acridines et les composés fluorones.

4. Procédé selon l'une quelconque des revendications précédentes, dans lequel le catalyseur de polymérisation est un composé amine.

5. Procédé selon l'une quelconque des revendications précédentes, dans lequel la solution comprend, en outre, un ou plusieurs ingrédients choisis parmi les colorants, les agents de réticulation, l'eau et les mélanges de ceux-ci.

6. Procédé selon l'une quelconque des revendications précédentes, dans lequel la lumière est appliquée sur la solution via un masque, de sorte à délimiter des zones d'intensité lumineuse distinctes.

7. Procédé selon l'une quelconque des revendications précédentes, dans lequel la lumière est appliquée selon une distribution d'intensité modulée spatialement.

8. Procédé selon l'une quelconque des revendications précédentes, dans lequel la solution comprend de l'acrylamide, un initiateur de photopolymérisation de formule suivante : 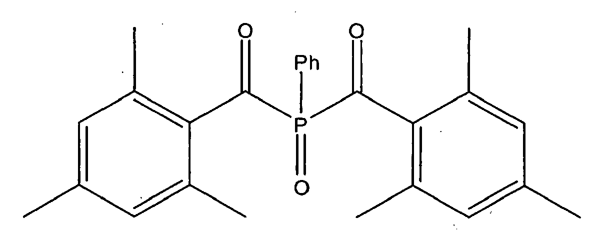 Ph désignant un groupe phényle,
de la n-propylamine en tant que catalyseur de polymérisation, de la N,N'-méthylènebisacrylamide en tant qu'agent de réticulation et de l'eau.

9. Procédé selon l'une quelconque des revendications précédentes, dans lequel la matrice de type hydrogel comprend au moins deux zones contiguës de rigidités distinctes présentant un gradient de rigidité égal à 1 kPa/µm.

10. Procédé selon l'une quelconque des revendications précédentes, comprenant, en outre, après l'étape de photopolymérisation, une étape de fonctionnalisation de la matrice par une protéine induisant une adhésion cellulaire via les intégrines.

11. Procédé d'adhésion cellulaire *in vitro* comprenant :
a) une étape de mise en oeuvre du procédé de préparation d'un matrice de type hydrogel tel que défini selon l'une quelconque des revendications 1 à 10;
b) une étape de dépôt sur la matrice obtenue à l'étape a) de cellules d'un ou plusieurs types, moyennant quoi les cellules d'au moins un des types susmentionnés adhérent en tout ou partie à certaines zones de la matrice de rigidité plus élevée que les zones contiguës.

12. Procédé de mise en forme cellulaire in vitro comprenant :
a) une étape de mise en oeuvre du procédé de préparation d'un matrice de type hydrogel tel que défini selon l'une quelconque des revendications 1 à 10;
b) une étape de dépôt sur la matrice obtenue à l'étape a) de cellules d'un ou plusieurs types, moyennant quoi les cellules d'au moins un des types susmentionnés se concentrent, en tout ou partie sur certaines zones de la matrice de rigidité plus élevée que les zones contiguës et adoptent la forme desdites zones de rigidité plus élevée.

13. Matrice de type hydrogel comprenant au moins deux zones contiguës de rigidités distinctes présentant un gradient de rigidité égal à 1 kPa/µm.

14. Matrice selon la revendication 13, dans laquelle une desdites deux zones contiguës présentant la rigidité la plus élevée se présente sous la forme d'un motif présentant une dimension inférieure à 100 µm.

15. Matrice selon la revendication 13 ou 14, qui est issue de la polymérisation de l'acrylamide et du N,N'-méthylènebisacrylamide.

## Patentansprüche

1. Verfahren zur Herstellung einer Matrix vom Hydrogeltyp, die wenigstens zwei zusammenhängende Zonen mit verschiedenen Festigkeiten umfaßt, umfassend einen Schritt der Photopolymerisation einer Lösung, umfassend eine oder mehrere polymerisierbare Verbindungen und einen Photopolymerisationsinitiator, durch Anwendung eines Lichts auf die gesamte Lösung und gemäß einer Intensität, die im Bereich von wenigstens zwei Zonen der Lösung unterschiedlich ist, woraus eine Hydrogelmatrix resultiert, die wenigstens zwei zusammenhängende Zonen mit unterschiedlichen Festigkeiten umfaßt, **dadurch gekennzeichnet, dass** der Schritt der Photopolymerisation in Anwesenheit eines Polymerisationskatalysators durchgeführt wird.

2. Verfahren nach Anspruch 1, bei dem die Matrix vom Hydrogeltyp aus einem Material ist, ausgewählt aus:
- Polyacrylamide;
- Polyethylenglykole, umfassend sich wiederholende Motive aus der Polymerisation von Acrylat- oder Methacrylatverbindungen;
- Polysacharide, gegebenenfalls modifiziert;
- (Ko)Polymere aus der Polymerisation von Diacrylat- und/oder (Meth)Acrylatverbindungen;
- Polyvinylalkohole, umfassend sich wiederholende Motive aus der Polymerisation von Acrylat- oder Methacrylatverbindungen;
- Dextrane, umfassend sich wiederholende Motive aus der Polymerisation von (Meth)Acrylatverbindungen;
- Polyfumarate von Propylen und deren Derivate; und
- Kombinationen hiervon.

3. Verfahren nach Anspruch 1 oder 2, bei dem der Photopolymerisationsinitiator ausgewählt ist aus den aromatischen Ketonen, den Akridinverbindungen und den Fluoronverbindungen.

4. Verfahren nach einem der vorhergehenden Ansprüche, bei dem der Polymerisationskatalysator eine Aminverbindung ist.

5. Verfahren nach einem der vorhergehenden Ansprüche, bei dem die Lösung ferner einen oder mehrere Inhaltsstoffe umfaßt, ausgewählt aus den Farbstoffen, den Vernetzungsagentien, Wasser und deren Mischungen.

6. Verfahren nach einem der vorhergehenden Ansprüche, bei dem das Licht auf die Lösung mittels einer Maske angewandt wird, derart, dass Zonen unterschiedlicher Lichtintensität begrenzt werden.

7. Verfahren nach einem der vorhergehenden Ansprüche, bei dem das Licht gemäß einer räumlich modulierten Intensitätsverteilung angewandt wird.

8. Verfahren nach einem der vorhergehenden Ansprüche, bei dem die Lösung Akrylamid umfaßt, einen Photopolymerisationsinitiator mit nachfolgender Formel: 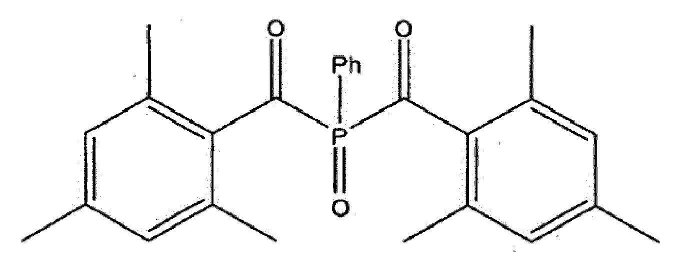 wobei Ph einen Phenylgruppe bezeichnet,
n-Propylamin als Polymerisationskatalysator, N, N'-Methylenbisacrylamid als Vernetzungsagens sowie Wasser.

9. Verfahren nach einem der vorhergehenden Ansprüche, bei dem die Matrix vom Hydrogeltyp wenigstens zwei zusammenhängende Zonen mit unterschiedlichen Festigkeiten umfaßt, die einen Festigkeitsgradienten gleich 1kPa/µm aufweisen.

10. Verfahren nach einem der vorhergehenden Ansprüche, ferner umfassend, nach dem Schritt der Photopolymerisation, einen Schritt der Funktionalisierung der Matrix durch ein Protein, das eine zelluläre Adhäsion über Integrine induziert.

11. Verfahren zur zellulären Adhäsion in vitro, umfassend:
a) einen Schritt der Durchführung des Verfahrens zur Herstellung einer Matrix vom Hydrogeltyp wie in einem der Ansprüche 1 bis 10 definiert;
b) einen Schritt des Aufbringens, auf der im Schritt a) erhaltenen Matrix, von Zellen eines oder mehrerer Typen, wodurch die Zellen wenigstens eines der genannten Typen vollständig oder teilweise an bestimmten Zonen der Matrix mit größerer Festigkeit als die zusammenhängenden Zonen anhaften.

12. Verfahren zur zellulären Formung in vitro, umfassend:
a) einen Schritt der Durchführung des Verfahrens zur Herstellung einer Matrix vom Hydrogeltyp wie in einem der Ansprüche 1 bis 10 definiert;
b) einen Schritt des Aufbringens, auf der im Schritt a) erhaltenen Matrix, von Zellen eines oder mehrerer Typen, wodurch sich die Zellen wenigstens eines der genannten Typen vollständig oder teilweise in bestimmten Zonen der Matrix mit größerer Festigkeit als die zusammenhängenden Zonen konzentrieren und die Form der Zonen mit größerer Festigkeit annehmen.

13. Matrix vom Hydrogeltyp, umfassend wenigstens zwei zusammenhängende Zonen mit unterschiedlichen Festigkeiten, die einen Festigkeitsgradienten gleich 1kPa/µm aufweisen.

14. Matrix nach Anspruch 13, bei der eine der zwei zusammenhängenden Zonen, die die größte Festigkeit aufweist, die Form eines Motivs aufweist, das eine Abmessung kleiner als 100µm aufweist.

15. Matrix nach Anspruch 13 oder 14, die aus der Polymerisation des Acrylamids und des N, N'-Metylenbisacrylamid stammt.

## Claims

1. Method for preparing a hydrogel type matrix comprising at least two contiguous zones with distinct stiffnesses comprising a step to photopolymerize a solution comprising one or several polymerizable compounds and a photopolymerization initiator, by application of light onto the entire solution and at a different intensity in at least two zones of the solution, as a result of which a hydrogel matrix is obtained comprising at least two contiguous zones with distinct stiffnesses, **characterized in that** the photopolymerization step is done in the presence of a polymerization catalyst.

2. Method according to claim 1, in which the hydrogel type matrix is made from a material chosen among:
- polyacrylamides;
- polyethyleneglycols containing repetitive patterns derived from polymerization of acrylate or methacrylate compounds;
- polysaccharides, possibly modified;
- (co)polymers derived from polymerization of diacrylate and/or (meth)acrylate compounds;
- polyvinyl alcohols containing repetitive patterns derived from polymerization of acrylate or methacrylate compounds;
- dextranes containing repetitive patterns derived from polymerization of (meth)acrylate compounds;
- polypropylene fumarates and derivatives of them; and
- combinations of these materials.

3. Method according to claim 1 or 2, in which the photopolymerization initiator is chosen from among aromatic ketones, acridine compounds and fluorone compounds.

4. Method according to any one of the previous claims, in which the polymerization catalyst is an amine compound.

5. Method according to any one of the previous claims, in which the solution also comprises one or several ingredients chosen among stains, cross-linking agents, water and mixtures of these ingredients.

6. Method according to any one of the previous claims, in which light is applied to the solution *through* a stencil, so as to delimit zones with distinct light intensity.

7. Method according to any one of the previous claims, in which light is applied according to a distribution with spatially modulated intensity.

8. Method according to any one of the previous claims, in which the solution comprises acrylamide, a photopolymerization initiator with the following formula: 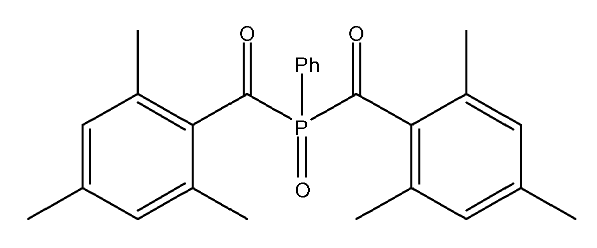 where Ph denotes a phenyl group,
n-propylamine as the polymerization catalyst, N,N'-methylenebisacrylamide as the cross-linking agent and water.

9. Method according to any one of the previous claims, in which the hydrogel type matrix comprises at least two contiguous zones with distinct stiffnesses with a stiffness gradient equal to 1 kPa/µm.

10. Method according to any one of the previous claims, also comprising a matrix functionalization step after the photopolymerization step, using a protein inducing cell adhesion through integrins.

11. *In vitro* cell adhesion method comprising:
a) a step to use the hydrogel type matrix preparation method as defined according to any one of claims 1 to 10;
b) a step in which cells of one or several types are deposited on the matrix obtained in step a), as a result of which the cells of at least one of the types mentioned above adhere entirely or partly to some zones of the matrix with higher stiffness than contiguous zones.

12. In vitro cell shaping method comprising:
a) a step to use the hydrogel type matrix preparation method as defined according to any one of claims 1 to 10;
b) a step to deposit cells of one or several types on the matrix obtained in step a), as a result of which the cells of at least one of the types mentioned above are concentrated entirely or partly on some zones of the matrix with higher stiffness than contiguous zones and adopt the shape of said higher stiffness zones.

13. Hydrogel type matrix comprising at least two contiguous zones with distinct stiffnesses having a stiffness gradient equal to 1 kPa/µm.

14. Matrix according to claim 13, in which one of said two contiguous zones with the highest stiffness is in the form of a pattern with dimensions smaller than 100 µm.

15. Matrix according to claim 13 or 14, which originates from polymerization of acrylamide and N,N'-methylenebisacrylamide.
